(19) Europäisches Patentamt
European Patent Office
Office européen des brevets

(11) Publication number: 0 019 365
A2

(12) **EUROPEAN PATENT APPLICATION**

(21) Application number: 80301260.8

(22) Date of filing: 18.04.80

(51) Int. Cl.³: C 07 C 87/127
C 07 C 85/08

(30) Priority: 11.05.79 GB 7916347

(43) Date of publication of application:
26.11.80 Bulletin 80/24

(84) Designated Contracting States:
BE CH DE FR GB IT LI NL SE

(71) Applicant: IMPERIAL CHEMICAL INDUSTRIES LIMITED
Imperial Chemical House Millbank
London SW1P 3JF(GB)

(72) Inventor: Hanson, Charles Barrie
7 Primrose Close
Guisborough Cleveland(GB)

(72) Inventor: Smith, Geoffrey Edwin Blaker
10 Castle Wynd Brass Castle Estate Nunthorpe
Middlesbrough Cleveland(GB)

(74) Representative: Martin, David Lincoln et al,
Imperial Chemical Industries Limited Legal Department
: Patents Thames House North Millbank
London SW1P 4QG(GB)

(54) **Long chain amine derivatives and process for their preparation.**

(57) The invention relates to alkylated amine compounds of formula:

$$R_1\text{-}\overset{H}{N}\text{-}R_2$$

where $R_1$ is selected from alkyl groups containing an odd number of carbon atoms in the range 7 to 17 carbon atoms and where $R_2$ comprises an alkyl group containing from 1 to 4 carbon atoms. In a preferred embodiment $R_1$ is a long chain alkyl group containing 13 or 15 carbon atoms, the groups being both straight chain and branched, the amount of branching being in the range 30 to 70%, and $R_2$ is a methyl group. The alkylated amine compounds are conveniently prepared to a purity of at least 95% by reaction of an aldehyde $R_1.CHO$ and an amine $R_2.NH_2$ in the presence of a suitable catalyst, for example Raney nickel or rhodium catalysts.

EP 0 019 365 A2

TITLE MODIFIED
see front page

Long Chain Amine Derivatives

THIS INVENTION relates to alkylated amines, to compositions based on these amines and to the manufacture and use of these amines and compositions.

Compositions based on long chain amines and their derivatives have been proposed for a wide variety of uses, for example as fabric softening agents and as emulsifiers. However, such prior art compositions have not always been entirely satisfactory, for example because of their limited purity or because they are difficult to handle.

We have now surprisingly found that certain long chain amine derivatives and compositions based on them have some advantageous properties and a wide variety of uses.

Accordingly, the present invention comprises alkylated amine compounds having the formula:

$$R_1 - \overset{\displaystyle H}{\overset{|}{N}} - R_2$$

where $R_1$ is selected from alkyl groups containing an odd number of carbon atoms in the range 7 to 17 carbon atoms, preferably 13 and 15 carbon atoms, and where $R_2$ is an alkyl group containing from 1 to 4 carbon atoms, preferably methyl.

In a preferred embodiment of this invention the substituent $R_1$ comprises a long chain alkyl group containing 13 or 15 carbon atoms, the groups being both straight chain and branched, the amount of branching being in the range 30 to 70% and $R_2$ is a methyl group.

In more preferred embodiments of this invention, the substituent $R_1$ is a long chain alkyl group containing 13 or 15 carbon atoms comprising approximately 65 to 75% $C_{13}$ groups with approximately 35 to 25% $C_{15}$ groups (these percentages being calculated on the total of long chain alkyl groups) with approximately 40 to 55 wt% straight chain and 60 to 45 wt% 2-alkyl branched chain where the 2-alkyl group is predominantly methyl. This derivative

is hereinafter referred to as "Synprolam" (Registered Trade Mark) 35M.

Alkylated amine compounds according to this invention are conveniently prepared by alkylation of an alkylamine, for example a monoalkylamine. A convenient route is by the reaction of an aldehyde of formula $R_1.CHO$ and an amine $R_2NH_2$ where $R_1$ and $R_2$ have the meanings hereinbefore described, in the presence of a suitable catalyst, for example a Raney nickel catalyst or a rhodium catalyst. It is believed that other hydrogenation catalysts would also be suitable for use in the process of this invention. By careful control of the proportions of aldehyde and amine used in the reaction, it is possible to obtain a product of at least 95% purity despite the presence in the product of the unsubstituted hydrogen atom. Secondary fatty methyl amines having 16 or 18 carbon atoms in the long chain alkyl group have been described but because of side reactions the purity of the products has been adversely affected and limited to, at most, about 85% purity. The presence of impurities in such products makes them much less acceptable commercially and the Applicants believe that the relatively high level of impurity occurs because these products are made by reaction of a long chain amine and a short chain aldehyde. In addition such $C_{16}/C_{18}$ products have been obtained in the form of a paste whereas the preferred product of this invention, "Synprolam" (Registered Trade Mark) 35M is obtained as a liquid and thereby is more easily handled.

The process of the present invention employs a short chain amine and a long chain aldehyde and the aldehyde is preferably selected from synthetic aldehydes, for example those made by a carbonylation process.

The products of this invention are useful chemical intermediates, for example in the preparation of salts, amides and other derivatives.

The compounds of this invention and their preparation are further illustrated in the following Examples:

EXAMPLE 1

Manufacture of "Synprolam" 35M

A mixture of $C_{13}$ and $C_{15}$ aldehydes (155 g/hr) and a mixture of monomethylamine (46.5 g/hr) and methanol (ca 200 ml/hr) were passed over 974g of a 1% w/w rhodium on alumina catalyst at 90 to 105°C under 180 atmospheres gauge hydrogen pressure. Excess monomethylamine and methanol solvent were removed under vacuum using a rotary evaporator. The remaining product (2864g) was distilled at 15 mm Hg pressure and the fraction distilling between 140 and 175°C (1967g) was collected. This fraction was analysed by mass spectrometry and found to contain mainly methyltridecylamine together with methylpentadecylamine. Infra-red and nuclear magnetic resonance spectra were also consistent with a mixture of linear and 2-methyl branched methyltridecylamines and methylpentadecylamines. By potentiometric titration, the product was shown to contain 95.5% secondary amine (as molecular weight 223).

EXAMPLE 2

Manufacture of "Synprolam" 35M

To 186.4g monomethylamine, 44g Raney nickel catalyst ("Nicat" 101 supplied by Joseph Crosfield and Sons Limited) in 280g methanol under 435 p.s.i.g. hydrogen pressure at 115°C, 412g of a mixture of $C_{13}$ to $C_{15}$ aldehyde was added over 2 hours. The composition of the aldehyde mixture was as follows

| | |
|---|---|
| $C_{11}$ aldehydes | 1.4% |
| $C_{13}$ aldehydes | 65.1% |
| $C_{15}$ aldehydes | 30.6% |
| Hydrocarbons | 1.0% |
| Heavy Ends | 1.9% |

The aldehydes consisted of linear, 2-methyl branched,

and other branched aldehydes in the approximate ratio 48:37:4 respectively. The mixture was allowed to react for a further 30 minutes at 115°C and 435 p.s.i.g. After cooling, filtering and removal of excess monomethylamine and methanol solvent under vacuum using a rotary evaporator, the product was analysed by potentiometric titration and shown to contain 72% secondary amine (as molecular weight 223) judged to be ($C_{13}$ to $C_{15}$ alkyl) amine, 4% primary amine (as molecular weight 209) and 5% tertiary amine (as molecular weight 415).

DLM/JH

Claims

1. Alkylated amine compounds having the formula:

$R_1 - \overset{H}{\overset{|}{N}} - R_2$ where $R_1$ is selected from alkyl groups containing an odd number of carbon atoms in the range 7 to 17 carbon atoms and where $R_2$ comprises an alkyl group containing from 1 to 4 carbon atoms.

2. Alkylated amine compounds as claimed in claim 1 in which the substituent $R_1$ comprises a long chain alkyl group containing 13 or 15 carbon atoms, the groups being both straight chain and branched, the amount of branching being in the range 30 to 70% and $R_2$ is a methyl group.

3. Alkylated amine compounds as claimed in claim 1 or 2 in which the substituent $R_1$ is a long chain alkyl group containing 13 or 15 carbon atoms comprising approximately 65 to 75% $C_{13}$ groups with approximately 35 to 25% $C_{15}$ groups (these percentages being calculated on the total of long chain alkyl groups) with approximately 40 to 55 wt% straight chain and 60 to 45 wt% 2-alkyl branched chain where the 2-alkyl group is predominantly methyl.

4. A process for the manufacture of an alkylated amine compound of formula

$R_1 - \overset{H}{\overset{|}{N}} - R_2$ where $R_1$ and $R_2$ are as defined in claim 1 which comprises catalytically reacting an aldehyde of formula $R_1.CHO$ and an amine of formula $R_2NH_2$.

5. A process as claimed in claim 4 in which the catalyst is a Raney nickel catalyst or a rhodium catalyst.

6. A process as claimed in claim 4 or 5 in which the aldehyde is a synthetic aldehyde made by a carbonylation process.